# EUROPEAN PATENT SPECIFICATION

(11) **EP 2 298 405 B1**
(45) Date of publication and mention of the grant of the patent: **27.05.2020**
(21) Application number: 10179409.7
(22) Date of filing: 20.06.2003
(51) Int. Cl.: A61M 5/32, A61M 25/06

(54) **Catheter and introducer needle assembly with needle shield**
Katheter und Einführnadelanordnung mit Nadelschutz
Cathéter et ensemble aiguille-introducteur doté d'une protection d'aiguille

(30) Priority: 20.06.2002 US 390499 P; 17.12.2002 US 320960
(43) Date of publication of application: 23.03.2011
(62) Divisional of application: 07117417.1
(73) Proprietor: Becton, Dickinson and Company, Franklin Lakes, NJ 07417-1880 (US)
(72) Inventor: Howell, Glade H., Sandy, UT 84094 (US); Harding, Weston F., Lehi, UT 84043 (US); Cindrich, Christopher N., Draper, UT 84020 (US); Sonderegger, Ralph, Farmington, UT 84025 (US); Frodsham, Joseph, Kaysville, UT 84037 (US)
(74) Representative: dompatent von Kreisler Selting Werner - Partnerschaft von Patent- und Rechtsanwälten mbB

(56) References cited:
- EP-A1- 0 554 841
- WO-A-01/93940
- US-A- 5 120 321
- US-A- 6 117 108

## Description

### BACKGROUND OF THE INVENTION

### 1. Field of the invention

The subject invention relates to a needle shield assembly constructed to safely shield the sharp distal tip of a needle, and restrict distal movement of the needle tip via a tilting or "canting" plate after the tip is shielded.

### 2. Background of the Invention

Intravenous (IV) catheters are used for infusing fluid, such as normal saline solution, various medicaments and total parenteral nutrition, into a patient or withdrawing blood from a patient. Peripheral IV catheters tend to be relatively short, and are on the order of about one and one-half inches in length. A common type of IV catheter is an over the needle peripheral IV catheter. As its name implies, an over the needle catheter is mounted over an introducer needle having a sharp distal tip. The catheter and the introducer needle are assembled so that the distal tip of the introducer needle extends beyond the distal tip of the catheter with the bevel of the needle facing up away from the patient's skin.

The catheter and introducer needle assembly are inserted at a shallow angle through the patient's skin into a peripheral blood vessel (i.e., a smaller blood vessel that is not connected directly to the heart but is one of the branches of the central blood vessels that is directly connected to the heart). In order to verify proper placement of the assembly in the blood vessel, the clinician confirms that there is flashback of blood in the needle and in a flashback chamber located at the proximal end of the needle. Typically, the flashback chamber is formed as part of the needle hub. Once proper placement is confirmed, the clinician applies pressure to the blood vessel by pressing down on the patient's skin near the distal tip of the introducer needle and the catheter. This finger pressure occludes further blood flow through the introducer needle. The clinician withdraws the introducer needle, leaving the catheter in place, and attaches a fluid-handling device to the catheter hub. Once the introducer needle is withdrawn from the catheter, it is deemed a "blood contaminated sharp" and must be properly handled.

In recent years, there has been great concern over the contamination of clinicians with a patient's blood and a recognition that "blood contaminated sharps" must be immediately disposed. This concern has arisen, in part, to reduce the risks associated with spreading diseases that can be transmitted by the exchange of body fluids from an infected person to another person. Thus, it is desirable to avoid contact with the body fluid of an infected person. Various needle shields have been developed. Generally, such needle shields work for their intended purpose but could be improved. For example, some needle shields are bulky, difficult to use or require special features or techniques to be operative.

US 6,117,108 describes a safety IV catheter that includes a unitary resilient needle guard received in a catheter hub. The needle guard comprises an elastic clamp through which the needle extends. When the needle is retracted from the catheter a distal portion of the guard snaps over the tip of the needle and covers the needle tip. The guard snaps into a position in which it is clamped onto the needle shaft and in which its distal wall blocks access to the needle tip. In an embodiment the guard comprises a canting portion having an opening with an edge that may lock the needle guard onto the needle shaft. A different embodiment does not comprise any canting member. The needle has a static feature having a diameter larger than that of the needle. In the shielded position the needle tip is locked between the bent and portions of the needle guard and proximally by the static feature abutting against a proximal wall of the needle guard.

WO 01/93940 A2 describes a catheter and introducer needle assembly with a needle shield. The needle shield includes a resilient clip having a proximal end with a hole. The needle has a static feature. When the needle is proximally withdrawn, the static feature moves behind an edge of the needle guard where it is clamped so that the needle tip is fixed within a tubular channel of a housing.

EP 0 554 841 A1 describes a needle shield assembly wherein the needle shaft comprises a static feature. A needle tip cover is slidably mounted to the needle shaft and engageable with the static feature to prevent its removal therefrom. The tip escape from the shield is restricted distally by a transverse wall portion of the needle tip cover and proximally by the restricted movement of the static feature through a shield proximal opening. A canting member is not provided.

US 5 120 321 discloses a disposable needle provided with a needle shield. The needle shield has a shroud with a canted bore region. The needle has a static feature thereon. Upon withdrawal of the needle through the shroud the needle tip enters the shroud and cannot escape anymore because a narrow bore region is axially offset from the canted bore region through which the needle shaft extends.

It is an object of the invention to provide a needle shield assembly having an integrated washer and a floating plate.

### SUMMARY OF THE INVENTION

The needle shield assembly of the invention is defined by claim 1.

In accord with one aspect of the invention an over the needle catheter assembly includes a catheter adapter and a needle. The needle has a diameter and a distal tip, slidingly disposed within the catheter adapter. A needle shield assembly is slidably mounted on the needle. The needle shield assembly has an open distal end and an open proximal end through which the needle passes. A rigid plate, referred to as a "canting plate", is disposed within the needle shield assembly and has an unactivated first position and an activated second position. In the second position, the canting plate restricts needle movement. Means for retaining canting plate are provided. The canting plate retention means is in communication with the canting plate and responsive to proximal movement of the needle, whereby, when the needle tip is housed within the needle shield assembly, the canting plate retention means is actuated, causing distal movement of the needle to urge the canting plate from the unactivated first position to the activated second position.

### BRIEF DESCRIPTION OF THE DRAWINGS

A preferred embodiment is illustrated in the drawings in which like reference numerals refer to like elements and in which:
Fig. 1A is a front perspective view of an embodiment of the invention in which the canting plate is actuated by an integral spring member shown in an unactuated condition;
Fig. 1B is a side cross-sectional view of the embodiment shown in Fig. 18A in an unactuated condition;
Fig. 1C is a front perspective view of the embodiment shown in Fig. 18A shown in an actuated condition;
Fig. 1D is a cut-away side view of the embodiment shown in Fig. 18A in an actuated condition.

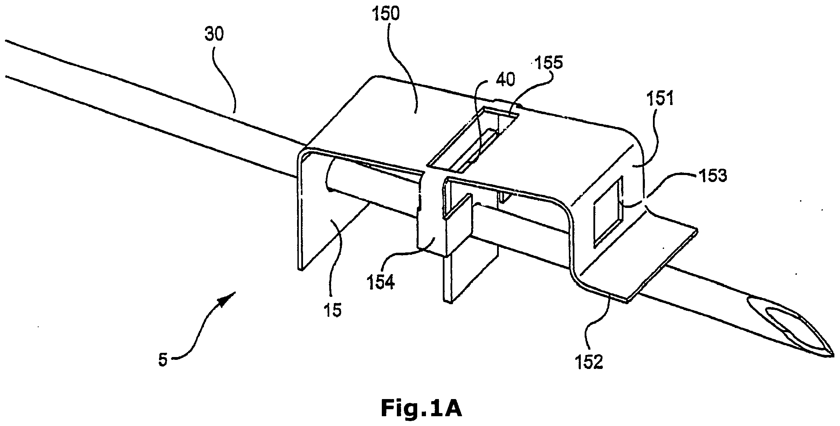

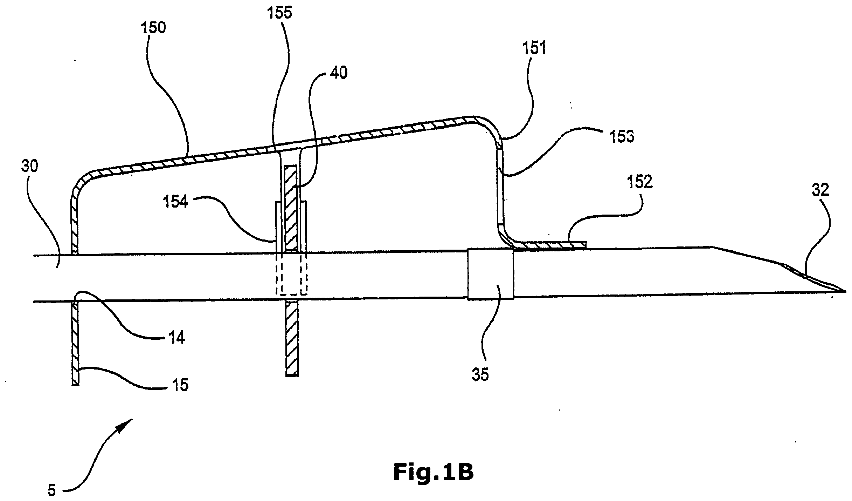

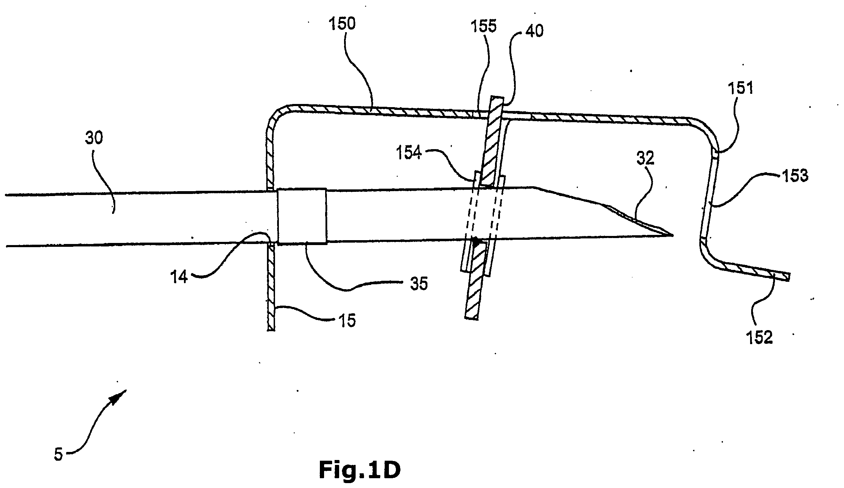

Referring now to Figs. 1A through 1D an implementation of the invention is depicted which may be designated as "Integrated Washer and Floating Plate". It includes a retention washer 15 integrally formed with an actuator arm 150. An opening 14 is disposed in the retention washer, a lip may be formed about the opening 14 to ease the passage of the needle and to ensure relatively perpendicular alignment between the retention washer and the needle. The actuator arm includes a front wall 151 and a slide plate 152. An aperture 153 is disposed in the front wall but may be eliminated in certain implementations. The canting plate 40 is maintained in position about the needle by a pair of u-shaped sleeves 154. The sleeves are in a relatively close fit with the canting plate 40 but not so dose that the canting plate cannot slide within the sleeves. Compare Fig. 1B and 1D. The u-shaped sleeves are themselves attached to the arm 150. An aperture 155 is disposed in the arm directly above the canting plate.

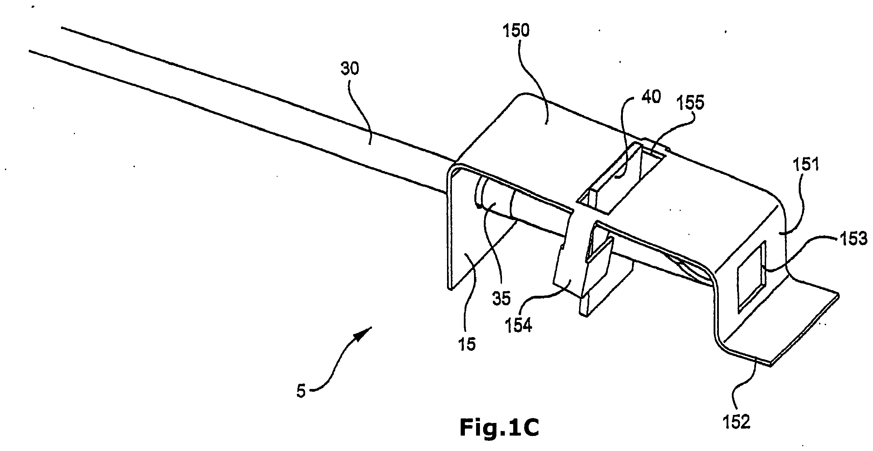

In the unactuated condition, as seen in Figs. 1A and 1B, the retention plate 15 and the arm 150 are flexed away from each other (that is, biased open) and maintained in this flexed condition by the presence of the needle 30 in the opening 14 of the retention washer, and the engagement of the needle with the plate 152. After insertion of the catheter into the patient's vein, the needle shield assembly 5 is moved toward the needle tip 32 (or, alternatively, the needle 30 is withdrawn through the needle shield assembly). As the needle moves proximally with respect to the needle shield assembly 5, the tip 32 of the needle passes beyond the slide plate 152 such that the arm 150 and retention plate 15 can return to their unbiased condition, rotating toward each other, as seen in Figs. 1C and 1D. In this unbiased or actuated condition, the u-shaped members 154 are displaced with respect to the retention washer 15 (specifically, the u-shaped members are rotated with respect to the retention plate). Compare Fig. 1B with Fig. 1D. As such, the canting plate 40 is also displaced with respect to the retention washer (and thus the needle). Effectively, the canting plate is tilted with respect to the needle and thereby engages the exterior of the needle. In the actuated condition, the top of the canting plate protrudes through the aperture 155 in the arm 150. It will be appreciated that the arm 150 could be designed such that such an aperture 155 would not be required but this would result in a larger needle shield assembly 5.

The preceding description is exemplary rather than limiting in nature. Variations and modifications to the disclosed examples may become apparent to those skilled in the art that do not necessarily depart from the purview and spirit of this invention. For example, implementations of the invention may be employed with other needles, such as anesthesia needles or syringes or blood sample collection sets. The scope of legal protection given to this invention can only be determined by studying the following claims.

## Claims

1. A needle shield assembly comprising:
a needle (30) having a first diameter and a distal tip (32);
a canting plate (40); and
a shield for covering the needle tip of the needle, said shield having a distal end and a proximal opening (14), said shield comprising an actuator arm (150) having on its proximal end an integrally formed proximal retention washer (15) with a proximal opening (14) and on its distal end an integrally formed front wall (151), said front wall being in engagement with the needle in an unactuated condition of the shield, wherein the shield covers the needle tip (32) when the needle is pulled sufficiently proximal, so that the shield takes an actuated condition when the front wall disengages from the needle;
**characterized by**
a static feature (35) on the needle having at least one second diameter larger than the needle first diameter, said proximal opening (14) of said shield, having a diameter smaller than said second diameter,
whereby tip escape from the shield is restricted distally by the canting of the canting plate (40) and proximally by the restricted movement of the second diameter through the shield proximal opening (14),
said shield further comprising first and second u-shaped members (154), wherein the canting plate (40) is configured to slide within said first and second u-shaped members, wherein the first and second u-shaped members are attached to the actuator arm for maintaining the canting plate in position about the needle, and configured to move between a first position in the actuated condition and a second position in the unactuated condition,
wherein in an unactuated condition the retention washer (15) and the actuator arm (150) are flexed away from each other and maintained in this flexed condition by the presence of the needle (30), and in the actuated condition the u-shaped sleeves (154) are displaced with respect to the retention washer (15) whereby the canting plate (40) is tilted with respect to the needle and thereby engages the exterior of the needle.

2. The needle shield assembly of claim 1 wherein the actuator arm (150) comprises its distal end a slide plate (152).

3. The needle shield assembly of claim 2 wherein the actuator arm (150) comprises an aperture (155) through which the top of the canting plate (40) protrudes in the actuated position.

## Patentansprüche

1. Nadelschutzanordnung mit:
einer Nadel (30), die einen ersten Durchmesser und eine distale Spitze (32) aufweist;
einer Verkantungsplatte (40); und
einem Schutzteil zum Bedecken der Nadelspitze der Nadel, wobei das Schutzteil ein distales Ende und eine proximale Öffnung (14) aufweist, wobei das Schutzteil einen Betätigungsarm (150) aufweist, der an dessen proximalen Ende eine einstückig ausgebildete proximale Rückhaltescheibe (15) mit einer proximalen Öffnung (14) und an dessen distalen Ende eine einstückig ausgebildete Vorderwand (151) aufweist, wobei die Vorderwand in einem unbetätigten Zustand des Schutzteils mit der Nadel zusammengreift, wobei das Schutzteil die Nadelspitz (32) bedeckt, wenn die Nadel ausreichend proximal gezogen wird, so dass das Schutzteil einen betätigten Zustand annimmt, wenn die Vorderwand von der Nadel ausrückt;
**gekennzeichnet durch**
eine statische Vorrichtung (35) auf der Nadel, die mindestens einen zweiten Durchmesser aufweist, der größer als der erste Durchmesser der Nadel ist, wobei die proximale Öffnung (14) des Schutzteils einen Durchmesser aufweist, der kleiner als der zweite Durchmesser ist,
wobei das Austreten der Spitze aus dem Schutzteil distal durch das Verkanten der Verkantungsplatte (40) und proximal durch die beschränkte Bewegung des zweiten Durchmessers durch die proximale Öffnung (14) des Schutzteils hindurch beschränkt ist,
wobei das Schutzteil ferner ein erstes und ein zweites U-förmiges Element (154) aufweist, wobei die Verkantungsplatte (40) dazu ausgebildet ist, in dem ersten und dem zweiten U-förmigen Element zu gleiten, wobei das erste und das zweite U-förmige Element an dem Betätigungsarm angebracht sind, um die Verkantungsplatte in Position um die Nadel herum zu halten, und wobei die Verkantungsplatte (40) dazu ausgebildet ist, sich zwischen einer ersten Position in dem betätigten Zustand und einer zweiten Position in dem unbetätigten Zustand zu bewegen,
wobei in einem unbetätigten Zustand die Rückhaltescheibe (15) und der Betätigungsarm (150) voneinander weg gebogen sind und durch das Vorhandensein der Nadel (30) in diesem gebogenen Zustand gehalten werden, und wobei in dem betätigten Zustand die U-förmigen Hülsen (154) relativ zu der Rückhaltescheibe (15) verschoben sind, wodurch die Verkantungsplatte (40) relativ zu der Nadel geneigt ist und dadurch mit der Außenseite der Nadel zusammengreift.

2. Nadelschutzanordnung nach Anspruch 1, wobei der Betätigungsarm (150) an dessen distalen Ende eine Gleitplatte (152) aufweist.

3. Nadelschutzanordnung nach Anspruch 2, wobei der Betätigungsarm (150) einen Ausschnitt (155) aufweist, durch den die Oberseite der Verkantungsplatte (40) in der betätigten Position hervorragt.

## Revendications

1. Ensemble de protection d'aiguille comprenant :
une aiguille (30) ayant un premier diamètre et une pointe distale (32) ;
une plaque inclinable (40) ; et
un protecteur pour couvrir la pointe d'aiguille de l'aiguille, ledit protecteur ayant une extrémité distale et une ouverture proximale (14), ledit protecteur comprenant un bras d'actionnement (150) ayant, sur son extrémité proximale, une rondelle de retenue proximale formée d'une seule pièce (15) avec une ouverture proximale (14) et sur son extrémité distale, une paroi avant formée d'une seule pièce (151), ladite paroi avant étant engagée avec l'aiguille dans un état non actionné du protecteur, où le protecteur couvre la pointe d'aiguille (32) lorsque l'aiguille est tirée de manière suffisamment proximale, de sorte que le protecteur prenne un état actionné lorsque la paroi avant se désengage de l'aiguille ;
**caractérisé par**
un élément statique (35) sur l'aiguille ayant au moins un deuxième diamètre supérieur au premier diamètre d'aiguille, ladite ouverture proximale (14) dudit protecteur, ayant un diamètre inférieur audit deuxième diamètre,
moyennant quoi la sortie de pointe du protecteur est limitée de manière distale par l'inclinaison de la plaque inclinable (40) et de manière proximale par le mouvement limité du deuxième diamètre à travers l'ouverture proximale de protecteur (14),
ledit protecteur comprenant en outre des premier et deuxième éléments en forme de u (154), où la plaque inclinable (40) est configurée pour coulisser dans lesdits premier et deuxième éléments en forme de u, où les premier et deuxième éléments en forme de u sont fixés au bras d'actionnement pour maintenir la plaque inclinable en position autour de l'aiguille, et configurée pour se déplacer entre une première position dans l'état actionné et une deuxième position dans l'état non actionné,
dans lequel, dans un état non actionné, la rondelle de retenue (15) et le bras d'actionnement (150) sont fléchies de manière à s'éloigner l'un(e) de l'autre et maintenus dans cet état fléchi par la présence de l'aiguille (30), et dans l'état actionné, les manchons en forme de u (154) se déplacent par rapport à la rondelle de retenue (15), moyennant quoi la plaque inclinable (40) est inclinée par rapport à l'aiguille et s'engage ainsi avec l'extérieur de l'aiguille.

2. Ensemble de protection d'aiguille de la revendication 1, dans lequel le bras d'actionnement (150) comprend sur son extrémité distale une plaque de coulissement (152).

3. Ensemble de protection d'aiguille de la revendication 2, dans lequel le bras d'actionnement (150) comprend un orifice (155) à travers lequel la partie supérieure de la plaque inclinable (40) fait saillie dans la position actionnée.
